# EUROPEAN PATENT APPLICATION

(11) **EP 3 492 485 A1**
(43) Date of publication of application: **05.06.2019**
(21) Application number: 17836569.8
(22) Date of filing: 11.05.2017
(51) Int. Cl.: C07J 63/00, C12P 15/00

(54) **METHOD FOR PRODUCING SOYA SAPONINS**

(30) Priority: 01.08.2016 JP 2016151196; 28.04.2017 JP 2017089440
(71) Applicant: Kao Corporation, Chuo-ku Tokyo 103-8210 (JP)
(72) Inventor: TSUNO, Yuhei, Tochigi 321-3497 (JP); FUJIMATSU, Teruhisa, Tochigi 321-3497 (JP)
(74) Representative: Vossius & Partner Patentanwälte Rechtsanwälte mbB
(86) International application number: PCT/JP2017/017845
(87) International publication number: WO 2018/025462

(57) **Abstract**

Provided is a method for producing soyasaponins inexpensively and simply without crushing seeds or plant bodies and without performing organic solvent treatment. The method for producing soyasaponins comprises bringing at least a part of a plant body of a leguminous plant into contact with an aqueous solution while culturing or cultivating the plant body using the aqueous solution; and collecting the aqueous solution or separating soyasaponins from the aqueous solution.

## Description

### Field of the Invention

The present invention relates to a method for producing soyasaponins.

### Background of the Invention

Soyasaponins are characteristic metabolites contained in leguminous plants, such as soybeans, and are widely used as emulsifiers in the food industry. Furthermore, in recent years, physiological active effects of soyasaponins, such as an anti-inflammatory effect, an anti-mutagenic effect, an anti-tumor effect, a hepatoprotective effect, and a pulmonary protective effect, are also drawing attention. As the method for obtaining soyasaponins, a method in which seeds of a leguminous plant (for example, soybean seeds) are crushed and defatted and then extracted with an organic solvent is typical. In addition, column purification is further performed as needed. Furthermore, Patent Literature 1 discloses a method in which a leguminous plant is subjected to crushing, acid treatment, alcohol extraction, and defatting and is then subjected to adsorption resin column treatment. Patent Literature 2 discloses a method in which soybean hypocotyl is extracted with alcohol and is applied to an anion exchange resin and the eluate is then adsorbed to a nonpolar synthetic adsorbent.

In extraction of soyasaponins from, for example, soybean seeds, generally, isoflavones are also simultaneously extracted. The content of isoflavones in a soybean extract is usually higher than that of soyasaponins. For example, in Non-Patent Literature 1, the ratio (mass ratio) of soyasaponins to isoflavones extracted from a plurality of soybean varieties was about 0.5 on average. Since contamination with isoflavones has a risk of causing side effects such as infertility, soyasaponins having a higher purity with a lower content of isoflavones are desired. Patent Literature 3 discloses a method in which an extract of defatted soybeans is adjusted to about pH 4.5 and centrifugated, and the residue is extracted with an alcohol aqueous solution. Patent Literature 3 states that the weight ratio of soyasaponins to isoflavone in a product was increased to 17.3 at the maximum by this method. However, the method described in Patent Literature 3 needs use of an organic solvent and operations such as, extraction, washing treatment, and centrifugation and is therefore complicated.

(Patent Literature 1) JP-A-01-275592
(Patent Literature 2) JP-A-2006-124324
(Patent Literature 3) JP-A-04-36242

(Non-Patent Literature 1) Goda et al., Food Hygiene and Safety Science, 43, 339-347, 2002

### Summary of the Invention

The present invention provides a method for producing soyasaponins comprising:
bringing at least a part of a plant body of a leguminous plant into contact with an aqueous solution while culturing or cultivating the plant body using the aqueous solution; and
collecting the aqueous solution or separating soyasaponins from the aqueous solution.

### Detailed Description of the Invention

Conventional methods for producing soyasaponins mainly use harvested seeds (beans) and therefore compete with food supply. In addition, the conventional methods for producing soyasaponins need heavy facilities for performing crushing plant bodies and organic solvent extraction and need disposal of extraction solvents and therefore have problems in cost and environmental aspects.

The present invention provides a method for producing soyasaponins inexpensively and simply without performing crushing of seeds or plant bodies and organic solvent treatment.

The present inventors have found that soyasaponins exude from growing plant bodies of leguminous plants. Based on this finding, the present inventors further found that soyasaponins can be produced inexpensively and simply by culturing or cultivating a leguminous plant and collecting the exudate without performing crushing of plant bodies or seeds and organic solvent extraction.

According to the present invention, soyasaponins can be produced inexpensively and simply. Since the method of the present invention does not need to crush the seeds or plant bodies of a leguminous plant and does not need to perform organic solvent extraction, it is possible to supply a leguminous plant useful as a food in parallel with the production of soyasaponins. Accordingly, the method of the present invention can also provide a high-value-added plant cultivation system.

The method for producing soyasaponins of the present invention comprises:
bringing at least a part of a plant body of a leguminous plant into contact with an aqueous solution while culturing or cultivating the plant body using the aqueous solution; and
collecting the aqueous solution or separating soyasaponins from the aqueous solution.

Examples of the "soyasaponins" produced by the method of the present invention include glycosides of which aglycone is soyasapogenol B (hereinafter, also referred to as group B), glycosides of which aglycone is soyasapogenol E (hereinafter, also referred to as group E), and glycosides of which aglycone is soyasapogenol A (hereinafter, also referred to as group A). The soyasaponins produced by the method of the present invention are at least one, any two or more, or all of the compounds selected from the group consisting of the soyasaponins of group B, group E, and group A above.

Examples of the soyasaponins of group B include glycosides in which a sugar residue is bound to the C-3 position of the aglycone of soyasapogenol B represented by the following Formula (I). Examples of the soyasaponins of group E include glycosides in which a sugar residue is bound to the C-3 position of the aglycone of soyasapogenol E represented by the following Formula (II). Examples of the soyasaponins of group A include glycosides in which a sugar residue is bound to the C-3 position of the aglycone of soyasapogenol A represented by the following Formula (III).

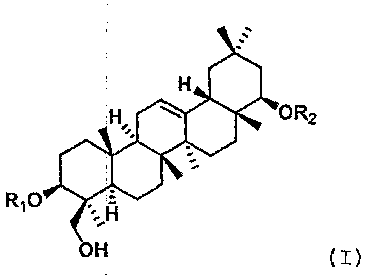

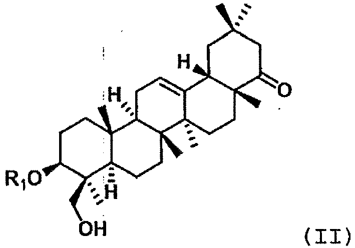

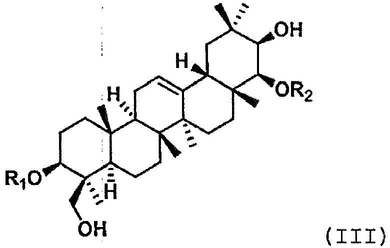

In Formulae (I) to (III), R₁'s each independently represent a sugar residue or a sugar chain; and R₂'s each independently represent a sugar residue, a sugar chain, or a hydrogen atom.

In Formulae (I) to (III), examples of the sugar residue represented by R₁ include glucuronic acid, galactose, glucose, rhamnose, and arabinose; examples of the sugar chain represented by R₁ include rhamnose (1→2) galactose (1→2) glucuronic acid (1→3); examples of the sugar residue represented by R₂ include arabinose, glucose (or 2,3,4,6-tetraacetyl form), and xylose (or 2,3,4-triacetyl form); and examples of the sugar chain represented by R₂ include glucose (1→3) arabinose (1→22).

Examples of the compound represented by Formula (I) preferably include soyasaponin I, soyasaponin II, and soyasaponin V, more preferably soyasaponin I. Preferred examples of the compound represented by Formula (II) include dehydrosoyasaponin I. Examples of the compound represented by Formula (III) preferably include soyasaponin A1 and soyasaponin A2, more preferably soyasaponin A2.

Preferably, the soyasaponins produced by the method of the present invention include the soyasaponins of group B and more preferably include the compounds represented by Formula (I).

The "leguminous plant" used in the method of the present invention may be any plant from which at least soyasaponins (group B) having the aglycone of soyasapogenol B as a basic skeleton exudes to the outside thereof. Examples of the leguminous plant used in the method of the present invention include Glycine plants, Vigna plants, Pisum plants, Medicago plants, Phaseolus plants, Vicia plants, Cicer plants, Neptunia plants, Trigonella plants, Lens plants, Arachis plants, and Psophocarpus plants. Among these plants, preferred examples are Glycine plants, Vigna plants, Pisum plants, Medicago plants, Phaseolus plants, Vicia plants, and Cicer plants. Among them, more preferred examples are Glycine plants, Vigna plants, Pisum plants, Medicago plants, and Vicia plants. Among them, further preferred examples are Glycine plants, Pisum plants, and Vicia plants. Among them, more preferred examples are Pisum plants and vicia plants. As an example of Glycine plants, a soybean (Glycine max) can be mentioned. As an example of Vigna plants, a mung bean (green gram) (Vigna radiata) can be mentioned. As an example of Pisum plants, pea (Tomyo) (Pisum sativum) can be mentioned. As an example of Medicago plants, lucerne (alfalfa) (Medicago sativa) can be mentioned. As an example of Phaseolus plants, a kidney bean (Phaseolus vulgaris) can be mentioned. As an example of Vicia plants, a broad bean (Vicia faba) can be mentioned. As an example of Cicer plants, a chickpea (Cicer arietinum) can be mentioned. As an example of Neptunia plants, water mimosa (Neptunia oleracea) can be mentioned. As an example of Trigonella plants, fenugreek (Trigonella foenum-graecum) can be mentioned. As an example of Lens plants, a lentil (Lens culinaris) can be mentioned. As an example of Arachis plants, peanut (Arachis hypogaea) can be mentioned. As an example of Psophocarpus plants, a winged bean (Psophocarpus tetragonolobus) can be mentioned. In the method of the present invention, any one or any two or more of the leguminous plants mentioned above can be used.

The "plant body" of a leguminous plant used in the present invention may be in any growing stage and may be in any state. Examples of the plant body include a seed; a germinating seed; a whole plant on the cotyledon development stage, the primary leaf development stage, the trifoliate leaf development stage, or the subsequent growth stage; a part of the seed, the germinating seed, or the whole plant; and a callus. Preferably, a plant body on or after the germination stage is used. More preferably, a plant body on the primary leaf development stage is used.

In the method of the present invention, a plant body of a leguminous plant is cultured or cultivated using an aqueous solution. A variety of cultivation techniques are encompassed as the technique of the culture or cultivation, and the technique can be appropriately selected according to the species or the growth stage of the plant as a subject. Examples of the technique of culture or cultivation include soilless cultivation, such as hydroponic cultivation, spray cultivation, sand cultivation, and gravel cultivation; soil cultivation; and drip fertigation. Among them, soilless cultivation is preferred, and hydroponic cultivation is more preferred.

As the aqueous solution used in the above culture or cultivation, a cultivation nutrient solution which can be used for culturing or cultivating leguminous plants, and preferably a cultivation nutrient solution which can be used for soilless cultivation of leguminous plants can be mentioned. Examples of such cultivation nutrient solution include a Murashige and Skoog (MS) medium, a Gamborg B5 medium, a Broughton & Dilworth (B&D) medium (Broughton and Dilworth, Biochem. J., 125, 1075-1080, 1971), known solution media, such as Enshi formulation, Otsuka formulation, and Hyponex, and water. Among these solutions, water or a B&D medium having a concentration adjusted according to the nutritional requirement of a plant are preferred. These aqueous solutions may contain minerals, such as nitrogen (N), phosphorus (P), potassium (K), calcium (Ca), magnesium (Mg), sulfur (S), iron (Fe), manganese (Mn), zinc (Zn), boron (B), copper (Cu), nickel (Ni), molybdenum (Mo), and chlorine (Cl). The composition of the aqueous solution is not particularly limited, and it is preferable to select a solution suitable for the plant as a subject and the cultivation system.

In the present invention, in the culture or cultivation above, at least a part of the plant body of a leguminous plant is in contact with the aqueous solution. The contact time between at least a part of the plant body and the aqueous solution is preferably 12 hours or more. The contact may be intermittent but is preferably continuous. The part of the plant body to be brought into contact with the aqueous solution is preferably a root (including a subterranean stem, a rhizome, a tuberous root, an adventitious root, etc.). Another part (e.g., a stem) may be in contact with the aqueous solution, together with the root. Accordingly, in a preferred embodiment of the present invention, the plant body of a leguminous plant is cultured or cultivated using the above-described cultivation nutrient solution in a state in which at least a part or the whole of the roots is immersed in the cultivation nutrient solution.

In a preferred embodiment, the plant body of a leguminous plant which is cultured or cultivated by the method of the present invention is a plant body having a root (for example, a germinating seed having a root; a whole plant on or after the cotyledon development stage; a part thereof including a root, a callus differentiated into a root, etc.). In the method of the present invention, although a plant body having a root may be cultured or cultivated from the beginning, for example, a seed, germinating seed, or callus not having a root yet may be cultured or cultivated to grow a root and may be then further cultured or cultivated.

From the viewpoint of stability of the cultivation environment, the culture or cultivation of a leguminous plant of the present invention is preferably performed indoors. The conditions for the culture or cultivation, such as temperature and illuminance, are not necessarily controlled, but for securing stable quality and productivity of soyasaponins, it is desirable to artificially control the cultivation environment in a closed facility (for example, a greenhouse or a plant factory).

During the above culture or cultivation, soyasaponins exude from the plant body of the leguminous plant and are released into the aqueous solution being in contact with at least a part of the plant body. In the present specification, the term "exudation" of a substance from a plant body means that the plant releases (is releasing) the substance from all or a part of the living body as a result of promotion of the xylem pressure, diffusion, or transport (i.e., secretion). Accordingly, according to the present invention, after the culture or cultivation for an appropriate period, the aqueous solution containing soyasaponins exuding from the plant body of a leguminous plant can be obtained.

In an embodiment of the present invention, after a plant body of a leguminous plant is cultured or cultivated using an aqueous solution and brought into contact with the aqueous solution, the aqueous solution containing soyasaponins exuding from the plant body is collected. The objective soyasaponins can be produced by separating the soyasaponins from the aqueous solution. The contact between the plant body of a leguminous plant and an aqueous solution, the collection of the aqueous solution containing the soyasaponins exuding from the plant body, and the separation of the objective soyasaponins from the aqueous solution may be performed continuously or by a batch system. The batch system may be a batch system in which all the aqueous solution is collected and is replaced with a fresh one or may be a semi-batch system in which a part of the aqueous solution is collected and replaced with a fresh one. In the case of the continuous system, for example, the aqueous solution may be circulated so as to continuously pass through a hydrophobic resin column, and the soyasaponins adsorbed to the column may be collected.

Examples of the means for separating soyasaponins from an aqueous solution include a method in which an aqueous solution is collected and filtrated to remove impurities and is then lyophilized. Alternatively, a method by performing liquid-liquid distribution of an aqueous solution with, for example, ethyl acetate or hexane or a method of separating soyasaponins from an aqueous solution using a hydrophobic resin as an adsorbent (JP-A-5-58903) can also be used. Examples of the hydrophobic resin include Diaion HP20 (Mitsubishi Chemical Corporation).

In another embodiment of the present invention, objective soyasaponins are directly separated from an aqueous solution being in contact with the plant body of a leguminous plant. For example, soyasaponins can be separated from an aqueous solution by charging a hydrophobic resin carrier as described above into the aqueous solution to adsorb the objective soyasaponins thereto while culturing or cultivating the plant body of a leguminous plant using the aqueous solution and collecting the carrier after a certain period of time. When the carrier is charged into the aqueous solution, the aqueous solution is preferably stirred for increasing the adsorption rate of the objective substance to the carrier.

A high-concentration soyasaponins powder can be obtained by further subjecting the objective soyasaponins separated from the aqueous solution to drying by heating, lyophilization, or the like as needed.

In yet another embodiment of the present invention, a collected aqueous solution containing soyasaponins exuding from the plant body of a leguminous plant can be directly used for an intended use as a solution containing soyasaponins, without performing operations such as separation of the soyasaponins. Separated soyasaponins also can be used for an intended use in the same way as in the above solution containing soyasaponins. For example, the aqueous solution containing soyasaponins and the separated soyasaponins can be used as an active ingredient for adding the physiologically active functions of soyasaponins to a variety of compositions, such as agricultural materials, food additives, detergents, medicines, and food.

According to the present invention, the mass ratio of soyasaponins to isoflavones in the collected aqueous solution above or a product containing soyasaponins obtained by separation from the aqueous solution is characteristically 2.0 or more, preferably from 2.0 to 20,000, more preferably from 4.0 to 20,000, further preferably from 17.3 to 20,000, further preferably from 20 to 20,000, further preferably from 30 to 20,000, further preferably from 40 to 20,000, further preferably from 100 to 20,000, further preferably from 200 to 20,000, further preferably from 500 to 20,000, further preferably from 800 to 20,000, and further preferably from 900 to 20,000; and more preferably the upper limit of the above-mentioned ranges is 11,000, more preferably 5,000, more preferably 3,000, and more preferably 1,000. Incidentally, the mass ratio of soyasaponins to isoflavones in conventional soybean seed extracts is about 0.5. Isoflavones have concerns about side effects such as infertility. It is possible by the present invention to provide a soyasaponins fraction having less contamination with isoflavones without using a complicated extraction procedure, column purification, and so on.

Examples of the isoflavones in the present invention include daidzein, genistein, glycitein, and glucoside forms and malonylated glucoside forms having these three isoflavones as aglycones, i.e., daidzin, genistin, glycitin, malonyldaidzin, malonylgenistin, and malonylglycitin.

The soyasaponins produced by the method of the present invention may include, in addition to the above-described soyasaponins of groups A, B, and E, soyasaponins being glycosides of which aglycone is soyasapogenol B and having 2,3-dihydro-2,5-dihydroxy-6-methyl-4H-pyran-4-one acetal-bonded to the 22-position (hereinafter, also referred to as group DDMP).

Examples of the soyasaponins of group DDMP include compounds represented by the following Formula (IV):

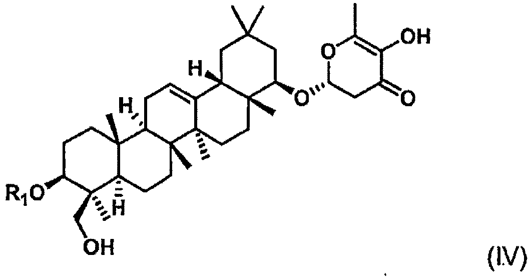

In Formula (IV), R₁ independently represents a sugar residue or a sugar chain.

In Formula (IV), examples of the sugar residue represented by R₁ include glucuronic acid, galactose, glucose, rhamnose, and arabinose; and examples of the sugar chain represented by R₁ include rhamnose (1→2) galactose (1→2) glucuronic acid (1→3).

Examples of the compound represented by Formula (IV) preferably include soyasaponin βg, soyasaponin βa, soyasaponin αa, and soyasaponin γg, and more preferably soyasaponin βg.

The contents of soyasaponin βg, soyasaponin βa, soyasaponin αa, and soyasaponin yg in the aqueous solution collected after used in the culture or cultivation of the plant body of a leguminous plant according to the method of the present invention are each preferably less than 0.05 µg/L.

Detection or quantitation of soyasaponins and isoflavones can be performed by a known method, for example, by thin-layer chromatography or liquid chromatography and using a mass spectrometer. Specific examples of the method of detection or quantitation include analysis by reversed phase high performance liquid chromatography (Jervis et al., J. Agric. Food Chem., 63, 9879-9887, 2015) and analysis by LC-MS.

In a preferred embodiment, the plant body of a leguminous plant used in the method of the present invention can be further cultured or cultivated or can be harvested after acquisition of the objective soyasaponins from the aqueous solution used in the culture or cultivation, without being killed or discarded. Accordingly, as a preferred embodiment of the method of the present invention, a method of performing soilless cultivation of a plant body until the best harvest period or the upper limit of the growth period and, in the process, appropriately collecting an objective substance from the nutrient solution (for example, see JP-A-60-92219 and JP-A-5-58903) can be employed. Here, the best harvest period is a time suitable for harvesting the plant body, and examples thereof include the vegetative growth stage, the flower bud formation stage, the flowering stage, the grain formation stage, and the grain maturation stage. Examples of the best harvest period in the case of using a leguminous plant as bean sprouts or sprouts include the cotyledon development stage including the period in which the hypocotyl extends and the primary leaf development stage.

As exemplary embodiments of the present invention, the following substances, production methods, uses, and methods are further disclosed in the present specification. However, the present invention is not limited to these embodiments.
[1] A method for producing soyasaponins comprising:
   bringing at least a part of a plant body of a leguminous plant into contact with an aqueous solution while culturing or cultivating the plant body using the aqueous solution; and
   collecting the aqueous solution or separating the soyasaponins from the aqueous solution.
[2] The method according to [1], wherein
   the culture or cultivation is
   preferably culture or cultivation by soilless cultivation, soil cultivation, or drip fertigation,
   more preferably culture or cultivation by hydroponic cultivation, spray cultivation, sand cultivation, gravel cultivation, or drip fertigation.
[3] The method according to [1] or [2], wherein preferably the soyasaponins are at least one selected from the group consisting of glycosides of which aglycone is soyasapogenol B, glycosides of which aglycone is soyasapogenol E, and glycosides of which aglycone is soyasapogenol A.
[4] The method according to any one of [1] to [3], wherein preferably the soyasaponins are at least one selected from the group consisting of compounds represented by the following Formulae (I) to (III): 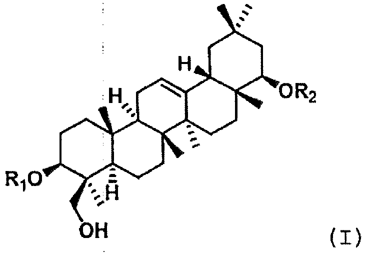 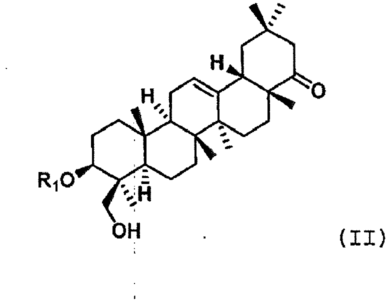 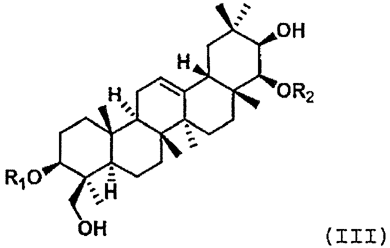 (in Formulae (I) to (III), R₁'s each independently represent a sugar residue or a sugar chain; and R₂'s each independently represent a sugar residue, a sugar chain, or a hydrogen atom).
[5] The method according to [4], wherein preferably, regarding R₁, the sugar residue is selected from the group consisting of glucuronic acid, galactose, glucose, rhamnose, and arabinose, and the sugar chain is rhamnose (1→2) galactose (1→2) glucuronic acid (1→3).
[6] The method according to [4] or [5], wherein preferably, regarding R₂, the sugar residue is selected from the group consisting of arabinose, glucose (or 2,3,4,6-tetraacetyl form), and xylose (or 2,3,4-triacetyl form), and the sugar chain is glucose (1→3) arabinose (1→22).
[7] The method according to [4], wherein
   the compound represented by Formula (I) is preferably selected from the group consisting of soyasaponin I, soyasaponin II, and soyasaponin V and is more preferably soyasaponin I;
   the compound represented by Formula (II) is preferably dehydrosoyasaponin I; and
   the compound represented by Formula (III) is preferably selected from the group consisting of soyasaponin A1 and soyasaponin A2 and is more preferably soyasaponin A2.
[8] The method according to any one of [1] to [7], wherein the mass ratio of soyasaponins to isoflavones in the collected aqueous solution or a product obtained by the separation is:
   preferably 2.0 or more;
   more preferably from 2.0 to 20,000, from 4.0 to 20,000, from 17.3 to 20,000, from 20 to 20,000, from 30 to 20,000, from 40 to 20,000, from 100 to 20,000, from 200 to 20,000, from 500 to 20,000, from 800 to 20,000, or from 900 to 20,000;
   further preferably from 2.0 to 11,000, from 4.0 to 11,000, from 17.3 to 11,000, from 20 to 11,000, from 30 to 11,0,00, from 40 to 11,000, from 100 to 11,000, from 200 to 11,000, from 500 to 11,000, from 800 to 11,000, or from 900 to 11,000;
   further preferably from 2.0 to 5,000, from 4.0 to 5,000, from 17.3 to 5,000, from 20 to 5,000, from 30 to 5,000, from 40 to 5,000, from 100 to 5,000, from 200 to 5,000, from 500 to 5,000, from 800 to 5,000, or from 900 to 5,000;
   further preferably from 2.0 to 3,000, from 4.0 to 3,000, from 17.3 to 3,000, from 20 to 3,000, from 30 to 3,000, from 40 to 3,000, from 100 to 3,000, from 200 to 3,000, from 500 to 3,000, from 800 to 3,000, or from 900 to 3,000; and
   further preferably from 2.0 to 1,000, from 4.0 to 1,000, from 17.3 to 1,000, from 20 to 1,000, from 30 to 1,000, from 40 to 1,000, from 100 to 1,000, from 200 to 1,000, from 500 to 1,000, from 800 to 1,000, or from 900 to 1,000.
[9] The method according to any one of [1] to [8], wherein the leguminous plant is:
   preferably at least one selected from the group consisting of Glycine plants, Vigna plants, Pisum plants, Medicago plants, and Vicia plants;
   more preferably at least one selected from the group consisting of Glycine plants, Pisum plants, and Vicia plants; and
   further preferably at least one selected from the group consisting of Pisum plants and Vicia plants.
[10] The method according to any one of [1] to [7], wherein preferably the leguminous plant is at least one selected from the group consisting of Cicer plants and Phaseolus plants.
[11] The method according to any one of [1] to [10], wherein the plant body is:
   preferably a seed; a germinating seed; a whole plant on the cotyledon development stage, the primary leaf development stage, the trifoliate leaf development stage, or the subsequent growth stage; a part of the seed, the germinating seed, or the whole plant; or a callus;
   more preferably a whole plant on or after the germination stage or a part thereof; and
   further preferably a whole plant of the primary leaf development stage or a part thereof.
[12] The method according to any one of [1] to [11], wherein preferably at least a part of the plant body in contact with the aqueous solution is a root.
[13] The method according to any one of [1] to [12], wherein preferably the aqueous solution is a solution medium or water.
[14] The method according to any one of [1] to [13], wherein preferably the contact time between at least a part of the plant body and the aqueous solution is 12 hours or more.
[15] The method according to any one of [1] to [14], preferably, further comprising separation of soyasaponins from the collected aqueous solution.
[16] The method according to any one of [1] to [15], wherein preferably the separation of soyasaponins from the aqueous solution is performed by lyophilization or using a hydrophobic resin.
[17] The method according to any one of [1] to [16], wherein the contents of soyasaponin βg, soyasaponin βa, soyasaponin αa, and soyasaponin yg in the collected aqueous solution are each less than 0.05 µg/L.
[18] Soyasaponins produced by the method according to any one of [1] to [17].
[19] The soyasaponins according to [18], preferably being in a form of a soyasaponins-containing aqueous solution or in a form of a powder.
[20] A soyasaponins-containing solution produced by the following steps of bringing at least a part of a plant body of a leguminous plant into contact with an aqueous solution while culturing or cultivating the plant body using the aqueous solution and collecting the aqueous solution.
[21] The soyasaponins-containing solution according to [20], wherein
   the culture or cultivation is:
   preferably culture or cultivation by soilless cultivation, soil cultivation, or drip fertigation; and
   more preferably culture or cultivation by hydroponic cultivation, spray cultivation, sand cultivation, gravel cultivation, or drip fertigation.
[22] The soyasaponins-containing solution according to [20] or [21], wherein preferably the soyasaponins are at least one selected from the group consisting of glycosides of which aglycone is soyasapogenol B, glycosides of which aglycone is soyasapogenol E, and glycosides of which aglycone is soyasapogenol A.
[23] The soyasaponins-containing solution according to any one of [20] to [22], wherein preferably the soyasaponins are at least one selected from the group consisting of compounds represented by the following Formulae (I) to (III): 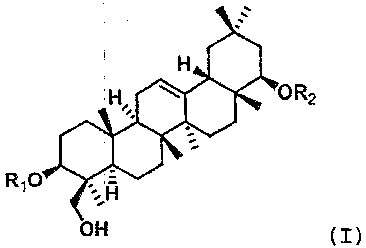 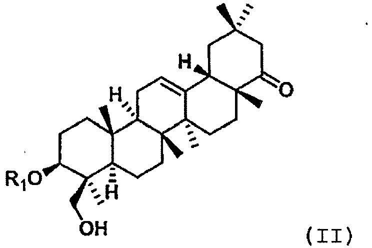 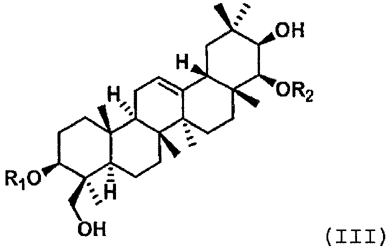 (in Formulae (I) to (III), R₁'s each independently represent a sugar residue or a sugar chain; and R₂'s each independently represent a sugar residue, a sugar chain, or a hydrogen atom).
[24] The soyasaponins-containing solution according to [23], wherein preferably, regarding R₁, the sugar residue is selected from the group consisting of glucuronic acid, galactose, glucose, rhamnose, and arabinose, and the sugar chain is rhamnose (1→2) galactose (1→2) glucuronic acid (1→3).
[25] The soyasaponins-containing solution according to [23] or [24], wherein preferably, regarding R₂, the sugar residue is selected from the group consisting of arabinose, glucose (or 2,3,4,6-tetraacetyl form), and xylose (or 2,3,4-triacetyl form), and the sugar chain is glucose (1→3) arabinose (1→22).
[26] The soyasaponins-containing solution according to [23], wherein
   the compound represented by Formula (I) is preferably selected from the group consisting of soyasaponin I, soyasaponin II, and soyasaponin V and is more preferably soyasaponin I;
   the compound represented by Formula (II) is preferably dehydrosoyasaponin I; and
   the compound represented by Formula (III) is preferably selected from the group consisting of soyasaponin A1 and soyasaponin A2 and is more preferably soyasaponin A2.
[27] The soyasaponins-containing solution according to any one of [20] to [26], wherein the mass ratio of soyasaponins to isoflavones is:
   preferably 2.0 or more;
   more preferably from 2.0 to 20,000, from 4.0 to 20,000, from 17.3 to 20,000, from 20 to 20,000, from 30 to 20,000, from 40 to 20,000, from 100 to 20,000, from 200 to 20,000, from 500 to 20,000, from 800 to 20,000, or from 900 to 20,000;
   further preferably from 2.0 to 11,000, from 4.0 to 11,000, from 17.3 to 11,000, from 20 to 11,000, from 30 to 11,000, from 40 to 11,000, from 100 to 11,000, from 200 to 11,000, from 500 to 11,000, from 800 to 11,000, or from 900 to 11,000;
   further preferably from 2.0 to 5,000, from 4.0 to 5,000, from 17.3 to 5,000, from 20 to 5,000, from 30 to 5,000, from 40 to 5,000, from 100 to 5,000, from 200 to 5,000, from 500 to 5,000, from 800 to 5,000, or from 900 to 5,000;
   further preferably from 2.0 to 3,000, from 4.0 to 3,000, from 17.3 to 3,000, from 20 to 3,000, from 30 to 3,000, from 40 to 3,000, from 100 to 3,000, from 200 to 3,000, from 500 to 3,000, from 800 to 3,000, or from 900 to 3,000; and
   further preferably from 2.0 to 1,000, from 4.0 to 1,000, from 17.3 to 1,000, from 20 to 1,000, from 30 to 1,000, from 40 to 1,000, from 100 to 1,000, from 200 to 1,000, from 500 to 1,000, from 800 to 1,000, or from 900 to 1,000.
[28] The soyasaponins-containing solution according to any one of [20] to [27], wherein the leguminous plant is:
   preferably at least one selected from the group consisting of Glycine plants, Vigna plants, Pisum plants, Medicago plants, and Vicia plants;
   more preferably at least one selected from the group consisting of Glycine plants, Pisum plants, and Vicia plants; and
   further preferably at least one selected from the group consisting of Pisum plants and Vicia plants.
[29] The soyasaponins-containing solution according to any one of [20] to [26], wherein preferably the leguminous plant is at least one selected from the group consisting of Cicer plants and Phaseolus plants.
[30] The soyasaponins-containing solution according to any one of [20] to [29], wherein the plant body is:
   preferably a seed; a germinating seed; a whole plant on the cotyledon development stage, the primary leaf development stage, the trifoliate leaf development stage, or the subsequent growth stage; a part of the seed, the germinating seed, or the whole plant; or a callus;
   more preferably a whole plant on or after the germination stage or a part thereof; and
   further preferably a whole plant of the primary leaf development stage or a part thereof.
[31] The soyasaponins-containing solution according to any one of [20] to [30], wherein preferably at least a part of the plant body in contact with the aqueous solution is a root.
[32] The soyasaponins-containing solution according to any one of [20] to [31], wherein preferably the aqueous: solution is a solution medium or water.
[33] The soyasaponins-containing solution according to any one of [20] to [32], wherein preferably the contact time between at least a part of the plant body and the aqueous solution is 12 hours or more.
[34] The soyasaponins-containing solution according to any one of [20] to [33], wherein the contents of soyasaponin βg, soyasaponin βa, soyasaponin αa, and soyasaponin yg are each less than 0.05 µg/L.

### Examples

The present invention will now be described in more detail based on Examples but is not limited thereto.

### Example 1

### (1. Cultivation of leguminous plant and collection of aqueous solution containing soyasaponins)

Soybean seeds (variety: Fukuyutaka (Nikko seed)) were sown on a urethane mat (M Hydroponic Research Co., Ltd.). The urethane mat was placed in a plastic vat containing tap water in the bottom. The seeds were subjected to submerged cultivation under fluorescent lamp lighting (16-hour light period/8-hour dark period, illuminance in the light period: about 10,000 lux) under conditions of a temperature of 25°C and a humidity of 50%. One week (budding time, VE), 2 weeks (primary leaf development stage, VC), and 3 weeks (first trifoliate leaf development stage, V1 stage) after the sowing, 3 samples were transferred in 50-mL centrifuge tubes (one sample in one tube) containing 50 mL of deionized water, respectively. On this occasion, the urethane portion was immersed and crumpled in deionized water for about 1 minute for substitution with water immediately before the transfer in the centrifuge tube and was then set so as to be completely immersed in the water in the centrifuge tube. The samples were left in a dark place for 16 hours under conditions of a temperature of 25°C and a humidity of 50% in a state in which the roots were immersed in water. An aqueous solution containing exudates from the roots was obtained by the operation above and was collected for producing soyasaponins.

### (2. Separation of soyasaponins from aqueous solution)

The aqueous solution containing soyasaponins obtained in Paragraph 1 was filtrated through a 0.45-µm pore size filter (Millipore Corporation, SLHV033RS) and was then subjected to solid phase extraction to concentrate the soyasaponins.

In the solid phase extraction, the carrier used was Sep-Pak C18 3cc Vac Cartridge, 200 mg Sorbent per Cartridge (Nihon Waters K.K.) (conditioning with 3 mL of methanol and washing with 3 mL of Milli-Q water (Merck KGaA)). The whole quantity of the filtered solution was added to the carrier for adsorption to a column, and soyasaponins were then separated by elution with 3 mL of methanol.

### (3. Identification and quantitation of soyasaponins)

The soyasaponins separated in Paragraph 2. were concentrated to dryness and were then redissolved in 200 µL of 50 v/v% methanol, and filtration with a filter was performed again to prepare a sample. Subsequently, the soyasaponins in the sample were analyzed by LC-MS. As reference standards, soyasaponin A1 (NP-000108, AnalytiCon Discovery GmbH) and A2 (NP-001666, AnalytiCon Discovery GmbH) were used as the soyasaponins of group A, soyasaponin I (P2505, Tokiwa Phytochemical Co., Ltd.), II (NP-000100, AnalytiCon Discovery GmbH), and V (P2506, Tokiwa Phytochemical Co., Ltd.) were used as the soyasaponins of group B, and dehydrosoyasaponin I (NP-017040, AnalytiCon Discovery GmbH) was used as the soyasaponin of group E. These reagents were analyzed by LC-MS to form a calibration curve.

### <LC-MS analysis conditions>

The HPLC apparatus and the mass spectrometer used were an Agilent 1260 Infinity LC system (Agilent Technologies, Inc.) and a Triple TOF 4600 system (AB Sciex Pte. Ltd.), respectively. The column used was CAPCELL CORE C18 (2.1 × 100 mm, 2.7 µm) (Shiseido Co., Ltd.). The eluents used were A: 0.1 v/v% formic acid solution and B: 0.1 v/v% formic acid-containing acetonitrile; the gradient conditions were 1 v/v% B for 0 to 0.1 minutes → 1 to 99.5 v/v% B for 0.1 to 13 minutes; and the flow rate was 0.5 mL/min. The ionization method was ESI (positive mode), and the measuring range was m/z 100-1550.

For each sample, soyasaponins A1 and A2 as the soyasaponins of group A, soyasaponins I, II, and V as the soyasaponins of group B, and dehydrosoyasaponin I as the soyasaponin of group E were identified from the retention time with each reagent, precise mass, and matching of MS/MS spectra. In addition, each soyasaponin in each sample was quantified from the calibration curve. The quantitative values were added together in each of groups A, B, and E (in group E, dehydrosoyasaponin I only) to determine the total amount (pmol or ng/plant) of soyasaponins of each group per individual plant or the total amount (pmol or ng/root) of soyasaponins of each group per fresh root mass. However, since standard reagents of the soyasaponins of group DDMP, i.e., soyasaponin βg, soyasaponin βa, soyasaponin αa, and soyasaponin γg, could not be obtained, they were approximately quantitated using a calibration curve formed with a reagent standard solution of soyasaponin I.

### (4. Quantitation of isoflavones)

As isoflavones, daidzein (D2668, Tokyo Chemical Industry Co., Ltd.), genistein (073-05531, FUJIFILM Wako Pure Chemical Corporation), and glycitein (NH010202, Nagara Science Co., Ltd.); glucoside forms having these three isoflavones as aglycones, i.e., daidzin (D3920, Tokyo Chemical Industry Co., Ltd.), genistin (73822, Sigma-Aldrich Japan LLC), and glycitin (ab142886, Abcam Biochemicals); and malonylated glucoside forms, i.e., malonyldaidzin (132-13821, FUJIFILM Wako Pure Chemical Corporation), malonylgenistin (136-13841, FUJIFILM Wako Pure Chemical Corporation), and malonylglycitin (139-13831, FUJIFILM Wako Pure Chemical Corporation), nine types in total, were used. These reagents were subjected to quantitative analysis by LC-MS in the same manner as that in Paragraph 3.

### (5. Results)

The results of analysis are shown in Tables 1, 2, and 3. The data in the tables indicate mean ± standard error of three samples. In the budding time (VE), about 140 pmol (about 150 ng) of soyasaponins (the total of group A, group B, and group E) exuded from the roots per soybean plant body. Similarly, about 2,090 pmol (about 2,070 ng) of soyasaponins were obtained in the primary leaf development stage (VC stage) and about 6,300 pmol (about 6,320 ng) of soyasaponins were obtained in the first trifoliate leaf development stage (V1 stage), from the roots as exudates per plant body (Table 1). When converted to soybean fresh root mass, in the budding time (VE), about 1,840 pmol (about 1960 ng) of soyasaponins (the total of group A, group B, and group E) exuded from the roots per 1 g of roots. Similarly, about 2,520 pmol (about 2,500 ng) of soyasaponins were obtained in the primary leaf development stage (VC stage) and about 4,490 pmol (about 4,490 ng) of soyasaponins were obtained in the first trifoliate leaf development stage (V1 stage), from the roots as exudates (Table 2). The mass ratio of soyasaponins to isoflavones in the exudate per soybean plant body was 5.7-fold in the VE stage, 18.9-fold in the VC stage, and 11.5-fold in the V1 stage (Table 3).

**[Table 1]**

| | Group | VE | VC | V1 |
|---|---|---|---|---|
| pmol/plant (ng/plant) | A | 104.59±31.85 (115.80±35.26) | 630.72±171.01 (702.35±193.36) | 2333.44±240.64 (2608.25±268.34) |
| | B | 32.85±8.67 (30.98±8.18) | 1285.89±271.39 (1204.31±253.52) | 3248.94±246.43 (3043.08±229.69) |
| | E | 3.74±3.74 (3.52±3.52) | 171.83±33.07 (161.71±31.12) | 712.54±53.21 (670.57±50.08) |
| | Total | 141.18±44.25 (150.30±46.95) | 2088.44±475.47 (2068.37±478.01) | 6294.92±540.28 (6321.90±548.10) |

**[Table 2]**

| | Group | VE | VC | V1 |
|---|---|---|---|---|
| pmol/g root (ng/g root) | A | 1380.43±426.98 (1528.41±472.75) | 754.02±145.21 (839.00±164.92) | 1593.68±126.03 (1782.09±142.18) |
| | B | 414.00±116.34 (390.44±109.72) | 1559.47±214.29 (1460.78±199.98) | 2357.21±429.18 (2207.79±401.59) |
| | E | 46.70±46.70 (43.95±43.95) | 210.18±25.50 (197.80±24.00) | 535.22±125.17 (503.69±117.79) |
| | Total | 1841.12±590.02 (1962.80±626.42) | 2523.67±385.00 (2497.58±388.90) | 4486.11±680.38 (4493.57±661.57) |

**[Table 3]**

| | | VE | VC | V1 |
|---|---|---|---|---|
| pmol/plant (ng/plant) | Total soyasaponin | 141.18±44.25 (150.30±46.95) | 2088.44±475.47 (2068.37±478.01) | 6294.92±540.28 (6321.90±548.10) |
| | Total isoflavone | 100.28±17.67 (26.21±4.61) | 423.85±36.93 (109.28±9.80) | 1919.25±412.30 (547.96±125.33) |
| Soyasaponin/isoflavone molar ratio (mass ratio) | | 1.41 (5.74) | 4.93 (18.93) | 3.28 (11.54) |

### Example 2

Seeds of kidney beans, broad beans, chickpeas, lucerne, pea, and mung beans (Nikko seed) were sown on urethane mats. These seeds were raised on a plastic vat filled with deionized water for 10 days after the sowing under fluorescent lamp lighting (16-hour light period/8-hour dark period, illuminance in the light period: about 10,000 lux) under conditions of a temperature of 25°C and a humidity of 50%. Five individuals of each plant species (but, in pea and mung beans, three individuals, respectively) were transferred in 50-mL centrifuge tubes. The individual plants were each placed such that the urethane portion for the roots fitted in the mouth of the centrifuge tube. To the centrifuge tube, 45 mL of a nutrient solution of Broughton & Dilworth (Broughton and Dilworth, Biochem. J., 125, 1075-1080, 1971) solution medium diluted to one-half, to which ammonium nitrate had been added to the final concentration of 0.5 mM, was added. The cultivation was performed in an artificial climate chamber (LPH-350SP, Nippon Medical & Chemical Instruments Co., Ltd.), the cultivation temperatures were 26°C in the light period and 20°C in the dark period, and the light conditions were 16-hour light period (about 15,000 lux) and 8-hour dark period. On the second or third day from the transfer to the centrifuge tubes (but, in Lucerne only, on the seventh day), five samples of each plant species (but, in pea and mung beans, three samples, respectively) were transferred to 50-mL centrifuge tubes filled with 45 mL of the nutrient solution and were left in a dark place for 16 hours in a state in which the roots were immersed in the nutrient solution. The resulting root exudate solution was subjected to filtration and solid phase extraction in the same manner as in Example 1 to concentrate the soyasaponins.

The resulting concentrate was redissolved in 200 µL of 50 v/v% methanol, and filtration with a filter was performed again to prepare a sample. Subsequently, the soyasaponins in the sample were analyzed and quantitated by LC-MS in the same manner as in Example 1. Regarding the soyasaponins of each group, the total amount (pmol or ng/plant) per individual plant or the total amount (pmol or ng/root) per 1 g of fresh root mass were determined.

The results of analysis are shown in Tables 4 and 5. The data in the tables indicate mean ± standard error of five samples (in pea and mung beans, three samples, respectively). In every case of six species of leguminous plants tested, soyasaponins (any of groups A, B, and E, or all of them) exuding from roots were obtained (Table 4). The mass ratio of soyasaponins to isoflavones greatly varied depending on the plant species, from 0.04-fold as the minimum to about 900-fold as the maximum (Table 5). Regarding pea showing the highest mass ratio of soyasaponins to isoflavones, the data of each individual (total amount (ng/plant) and mass ratio per individual) are shown in Table 6.

**[Table 4]**

| | Group | Kidney bean | Broad bean | Chickpea | Lucerne | Pea | Mung bean |
|---|---|---|---|---|---|---|---|
| pmol/plant (ng/plant) | A | 0.00 ±0.00 (0.00 ±0.00) | 0.00 ±0.00 (0.00 ±0.00) | 0.00 ±0.00 (0.00 ±0.00) | 0.00 ±0.00 (0.00 ±0.00) | 0.00 ±0.00 (0.00 ±0.00) | 0.00 ±0.00 (0.00 ±0.00) |
| | B | 17.67 ±2.51 (16.89 ±2.39) | 436.50 ±98.21 (411.66 ±92.62) | 4.90 ±0.97 (4.62 ±0.92) | 158.09 ±57.08 (147.17 ±52.79) | 5609.87 ±751.43 (3807.31 ±494.21) | 4966.44 ±922.89 (3149.41 ±494.68) |
| | E | 0.00 ±0.00 (0.00 ±0.00) | 90.00 ±21.99 (84.70 ±20.69) | 0.00 ±0.00 (0.00 ±0.00) | 26.10 ±4.89 (24.56 ±4.60) | 1576.70 ±281.28 (1483.83 ±264.72) | 3346.52 ±525.64 (3149.41 ±494.68) |
| pmol/g root (ng/g root) | A | 0.00 ±0.00 (0.00 ±0.00) | 0.00 ±0.00 (0.00 ±0.00) | 0.00 ±0.00 (0.00 ±0.00) | 0.00 ±0.00 (0.00 ±0.00) | 0.00 ±0.00 (0.00 ±0.00) | 0.00 ±0.00 (0.00 ±0.00) |
| | B | 22.93 ±4.09 (21.92 ±3.91) | 355.93 ±91.34 (335.68 ±86.15) | 14.69 ±5.96 (13.85 ±5.62) | 2489.97 ±737.54 (2319.96 ±680.96) | 6420.62 ±617.25 (6014.04 ±576.81) | 6420.62 ±617.25 (5443.64 ±1225.65) |
| | E | 0.00 ±0.00 (0.00 ±0.00) | 73.59 ±20.13 (69.26 ±18.95) | 0.00 ±0.00 (0.00 ±0.00) | 435.64 ±46.12 (409.98 ±43.40) | 2483.00 ±345.57 (2336.73 ±325.22) | 2482.98 ±943.59 (3631.73 ±888.01) |

**[Table 5]**

| | | Kidney bean | Broad bean | Chickpea | Lucerne | Pea | Mung bean |
|---|---|---|---|---|---|---|---|
| pmol/ plant (ng/plant) | Total soyasaponin | 17.67 ±2.51 (16.89 ±2.39) | 526.50 ±120.19 (496.36 ±113.31) | 4.90 ±0.97 (4.62 ±0.92) | 184.19 ±61.97 (171.74 ±57.39) | 7186.57 ±1032.71 (5291.13 ±758.92) | 8312.95 ±1448.54 (7811.31 ±989.31) |
| | Total isoflavone | 145.49 ±40.70 (39.54 ±11.21) | 47.88 ±44.78 (12.32 ±11.51) | 473.47 ±138.17 (128.15 ±37.52) | 145.81 ±27.69 (37.13 ±7.06) | 14.69 ±11.12 (5.85 ±4.90) | 4551.62 ±2539.4 (1198.18 ±663.97) |
| Soyasaponin/isoflavone molar ratio (mass ratio) | | 0.12 (0.43) | 11.00 (40.30) | 0.01 (0.04) | 1.26 (4.62) | 489.22 (904.60) | 1.83 (6.52) |

**[Table 6]**

| | | Pea-1 | Pea-2 | Pea-3 | Pea (average) |
|---|---|---|---|---|---|
| ng/plan | Total soyasaponin | 4890.22 | 4273.49 | 6709.7 | 5291.13 |
| | Total isoflavone | 14.78 | 0.40 | 2.37 | 5.85 |
| Soyasaponin/isoflavone molar ratio | | 330.83 | 10797.08 | 2831.1 | 904.6 |

### Example 3

The compositions of compounds of the aqueous solutions containing exudates from soybean roots obtained in Example 1 (1. Cultivation of leguminous plant and collection of aqueous solution containing soyasaponins), i.e., the soyasaponins solutions of VE stage soybeans, VC stage soybeans, and V1 stage soybeans were measured by LC-MS in the same manner as in Example 1 (detection limit: 0.05 µg/L). However, since standard reagents of the soyasaponins of group DDMP could not be obtained, they were approximately quantitated using a calibration curve formed with a reagent standard solution of soyasaponin I. The results are shown in Table 7. The numerical values in Table 7 indicate mean ± standard error (n = 5), and the unit is µg/L. In the root exudate solutions of soybeans, the soyasaponins of groups A, B, and E were detected, but the soyasaponins of group DDMP were not detected.

**[Table 7]**

| µg/L | Soyasaponin solution | | |
|---|---|---|---|
| | VE stage soybean | VC stage soybean | V1 stage soybean |
| Soyasaponin I | 0.7±0.2 | 17.9±3.4 | 42.4±2.5 |
| Soyasaponin II | 0.0±0.0 | 6.8±1.8 | 17.9±2.1 |
| Soyasaponin V | 0.0±0.0 | 2.1±0.4 | 7.3±0.5 |
| Soyasaponin A1 | 0.0±0.0 | 0.7±0.7 | 4.3±0.3 |
| Soyasaponin A2 | 2.6±0.8 | 14.9±3.6 | 53.7±5.6 |
| Dehydrosoyasaponin I | 0.1±0.1 | 3.6±0.7 | 14.9±1.1 |
| Daidzein | 0.4±0.1 | 2.2±0.2 | 8.8±1.7 |
| Genistein | 0.1±0.0 | 0.1±0.0 | 0.4±0.1 |
| Glycitein | 0.0±0.0 | 0.1±0.0 | 0.2±0.1 |
| Daidzin | 0.0±0.0 | 0.0±0.0 | 0.8±0.3 |
| Genistin | 0.0±0.0 | 0.0±0.0 | 0.1±0.0 |
| Glycitin | 0.0±0.0 | 0.0±0.0 | 0.0±0.0 |
| Malonyldaidzin | 0.0±0.0 | 0.0±0.0 | 1.8±0.6 |
| Malonylgenistin | 0.0±0.0 | 0.0±0.0 | 0.1±0.1 |
| Malonylglycitin | 0.0±0.0 | 0.0±0.0 | 0.0±0.0 |
| Soyasaponin βg* | 0.0±0.0 | 0.0±0.0 | 0.0±0.0 |
| Soyasaponin βa* | 0.0±0.0 | 0.0±0.0 | 0.0±0.0 |
| Soyasaponin αa* | 0.0±0.0 | 0.0±0.0 | 0.0±0.0 |
| Soyasaponin γg* | 0.0±0.0 | 0.0±0.0 | 0.0±0.0 |

| | | | |
|---|---|---|---|
| *: Group DDMP soyasaponin (approximation) | | | |

### Example 4

The compositions of compounds of the root exudate solutions of kidney beans, broad beans, chickpeas, lucerne, pea, and mung beans obtained in Example 2, i.e., soyasaponin solutions of kidney beans, broad beans, chickpeas, lucerne, pea, and mung beans were measured by LC-MS in the same manner as in Example 1 (detection limit: 0.05 µg/L). However, since standard reagents of the soyasaponins of group DDMP could not be obtained, they were approximately quantitated using a calibration curve formed with a reagent standard solution of soyasaponin I. The results are shown in Table 8. The numerical values in Table 8 indicate mean ± standard error (n = 3), and the unit is µg/L. In the root exudate solutions of leguminous plants other than soybeans, soyasaponins of group B or groups B and E were detected. In contrast, no soyasaponins of group DDMP were detected.

**[Table 8]**

| µg/L | Soyasaponin solution | | | | | |
|---|---|---|---|---|---|---|
| | Kidney bean | Broad bean | Chickpea | Lucerne | Pea | Mung bean |
| Soyasaponin I | 0.1±0.0 | 9.2±2.1 | 0.1±0.0 | 2.0±0.5 | 66.5±9.2 | 88.2±9.2 |
| Soyasaponin II | 0.0±0.0 | 0.0±0.0 | 0.0±0.0 | 1.3±0.7 | 17.6±1.6 | 15.1±1.7 |
| Soyasaponin V | 0.3±0.0 | 0.0±0.0 | 0.0±0.0 | 0.0±0.0 | 0.4±0.1 | 0.4±0.1 |
| Soyasaponin A1 | 0.0±0.0 | 0.0±0.0 | 0.0±0.0 | 0.0±0.0 | 0.0±0.0 | 0.0±0.0 |
| Soyasaponin A2 | 0.0±0.0 | 0.0±0.0 | 0.0±0.0 | 0.0±0.0 | 0.0±0.0 | 0.0±0.0 |
| Dehydrosoyasaponin I | 0.0±0.0 | 1.9±0.5 | 0.0±0.0 | 0.6±0.1 | 33.0±5.9 | 70.0±11.0 |
| Daidzein | 0.5±0.1 | 0.2±0.2 | 0.0±0.0 | 0.8±0.2 | 0.0±0.0 | 22.9±12.9 |
| Genistein | 0.2±0.1 | 0.1±0.1 | 2.8±0.8 | 0.0±0.0 | 0.0±0.0 | 0.9±0.4 |
| Glycitein | 0.0±0.0 | 0.0±0.0 | 0.0±0.0 | 0.0±0.0 | 0.0±0.0 | 1.4±0.8 |
| Daidzin | 0.1±0.0 | 0.0±0.0 | 0.0±0.0 | 0.0±0.0 | 0.0±0.0 | 0.2±0.1 |
| Genistin | 0.0±0.0 | 0.0±0.0 | 0.0±0.0 | 0.0±0.0 | 0.0±0.0 | 0.0±0.0 |
| Glycitin | 0.0±0.0 | 0.0±0.0 | 0.0±0.0 | 0.0±0.0 | 0.0±0.0 | 0.0±0.0 |
| Malonyldaidzin | 0.0±0.0 | 0.0±0.0 | 0.0±0.0 | 0.0±0.0 | 0.1±0.1 | 1.3±0.5 |
| Malonylgenistin | 0.0±0.0 | 0.0±0.0 | 0.0±0.0 | 0.0±0.0 | 0.0±0.0 | 0.0±0.0 |
| Malonylglycitin | 0.0±0.0 | 0.0±0.0 | 0.0±0.0 | 0.0±0.0 | 0.0±0.0 | 0.0±0.0 |
| Soyasaponin βg* | 0.0±0.0 | 0.0±0.0 | 0.0±0.0 | 0.0±0.0 | 0.0±0.0 | 0.0±0.0 |
| Soyasaponin βa* | 0.0±0.0 | 0.0±0.0 | 0.0±0.0 | 0.0±0.0 | 0.0±0.0 | 0.0±0.0 |
| Soyasaponin αa* | 0.0±0.0 | 0.0±0.0 | 0.0±0.0 | 0.0±0.0 | 0.0±0.0 | 0.0±0.0 |
| Soyasaponin γg* | 0.0±0.0 | 0.0±0.0 | 0.0±0.0 | 0.0±0.0 | 0.0±0.0 | 0.0±0.0 |

| | | | | | | |
|---|---|---|---|---|---|---|
| *: Group DDMP soyasaponin (approximation) | | | | | | |

### Comparative Example 1

Soybean (Fukuyutaka) seeds were crushed with, for example, a mortar. 10 mg thereof were subjected to homogenization extraction (5 min, 20 Hz (ball mill MM400, Retsch GmbH)) with 1 mL of 80 v/v% methanol. The extract was filtrated through a 0.45-µm filter. The composition of compounds in the filtrate was measured by LC-MS with the same method as that in the root exudate solution. However, since standard reagents of the soyasaponins of group DDMP were not able to be obtained, they were approximately quantitated using a calibration curve formed with a reagent standard solution of soyasaponin I. The results are shown in Table 9. The unit of the numerical values in Table 9 is weight of each compound per dry seed weight, µg/g. Although no soyasaponins of group A were detected in the soybean seeds, soyasaponins of group B and group E were detected. In addition, the soyasaponins of group DDMP were detected.

**[Table 9]**

| µg/g | Fukuyutaka seed |
|---|---|
| Soyasaponin I | 88.3 |
| Soyasaponin II | 85.9 |
| Soyasaponin V | 12.9 |
| Soyasaponin A1 | 0.0 |
| Soyasaponin A2 | 0.0 |
| Dehydrosoyasaponin I | 35.1 |
| Daidzein | 6.7 |
| Genistein | 7.1 |
| Glycitein | 0.2 |
| Daidzin | 187.2 |
| Genistin | 278.5 |
| Glycitin | 10.4 |
| Malonyldaidzin | 446.2 |
| Malonylgenistin | 1250.3 |
| Malonylglycitin | 27.3 |
| Soyasaponin βg* | 398.0 |
| Soyasaponin βa* | 88.0 |
| Soyasaponin αa* | 9.8 |
| Soyasaponin γg* | 113.2 |

| | |
|---|---|
| *: Group DDMP soyasaponin (approximation) | |

### Comparative Example 2

Soybean roots obtained in Example 1 (1. Cultivation of leguminous plant and collection of aqueous solution containing soyasaponins) were lyophilized and were subjected to homogenization extraction (5 min, 20 Hz (ball mill MM400, Retsch GmbH)) with 50 v/w times 80 v/v% methanol. That is, 10 mg of dried roots were weighed and were extracted with 500 µL of a solvent. The extract was filtrated through a 0.45-µm filter. The filtrate was appropriately diluted with 80 v/v% methanol. The composition of compounds in the filtrate was measured by LC-MS with the same method as that in the root exudate solution. However, since standard reagents of the soyasaponins of group DDMP could not be obtained, they were approximately quantitated using a calibration curve formed with a reagent standard solution of soyasaponin I. The results are shown in Table 10. The numerical values in Table 10 indicate mean ± standard error (n = 5), and the unit is weight of each compound per dry root weight, µg/g. In the root extracts of VE stage to V1 stage soybeans, the soyasaponins of group DDMP were detected in addition to the soyasaponins of groups A, B, and E.

**[Table 10]**

| µg/g | VE stage soybean | VC stage soybean | V1 stage soybean |
|---|---|---|---|
| Soyasaponin I | 1574.3±138.9 | 1909.8±61.8 | 1602.8±65.3 |
| Soyasaponin II | 683.6±114.1 | 999.5±64.3 | 1047.5±74.1 |
| Soyasaponin V | 243.4±18.1 | 245.9±12.4 | 196.7±4.2 |
| Soyasaponin A1 | 0.0±0.0 | 0.0±0.0 | 0.0±0.0 |
| Soyasaponin A2 | 377.8±24.8 | 660.7±48.5 | 664.7±69.5 |
| Dehydrosoyasaponin I | 407.9±43.6 | 1086.3±58.0 | 755.6±30.4 |
| Daidzein | 437.6±37.0 | 3418.6±386.4 | 3170.8±158.4 |
| Genistein | 86.5±9.2 | 492.5±66.8 | 282.6±37.2 |
| Glycitein | 0.8±0.2 | 1.9±0.3 | 12.6±1.0 |
| Daidzin | 58.6±7.6 | 544.0±68.8 | 1525.3±206.6 |
| Genistin | 17.6±1.4 | 66.8±10.6 | 153.1±22.1 |
| Glycitin | 0.2±0.1 | 0.4±0.1 | 12.1±2.9 |
| Malonyldaidzin | 261.7±29.7 | 4821.0±668.1 | 16893.4±2131.9 |
| Malonylgenistin | 75.2±8.9 | 984.1±147.5 | 3554.0±565.6 |
| Malonylglycitin | 1.3±0.4 | 8.4±1.3 | 151.5±37.7 |
| Soyasaponin βg* | 130.3±0.6 | 27.8±2.5 | 394.1±107.5 |
| Soyasaponin βa* | 7.9±1.8 | 0.0±0.0 | 53.3±17.8 |
| Soyasaponin αa* | 0.0±0.0 | 0.0±0.0 | 6.7±2.1 |
| Soyasaponin γg* | 5.0±0.9 | 0.0±0.0 | 17.8±5.0 |

| | | | |
|---|---|---|---|
| *: Group DDMP soyasaponin (approximation) | | | |

### Comparative Example 3

Roots of each leguminous plant (other than soybeans) obtained in Example 1 (1. Cultivation of leguminous plant and collection of aqueous solution containing soyasaponins) were lyophilized and were subjected to homogenization extraction (5 min, 20 Hz (ball mill MM400, Retsch GmbH)) with 250 v/w times 80 v/v% methanol. That is, 3 mg of dried roots were weighed and were extracted with 750 µL of a solvent. The extract was filtrated through a 0.45-µm filter. The filtrate was appropriately diluted with 80 v/v% methanol. The composition of compounds in the filtrate was measured by LC-MS with the same method as that in the root exudate solution. However, since standard reagents of the soyasaponins of group DDMP could not be obtained, they were approximately quantitated using a calibration curve formed with a reagent standard solution of soyasaponin I. The results are shown in Tables 11 and 12. The numerical values in Tables 11 and 12 indicate mean ± standard error (n = 3), and the unit is weight of each compound per dry root weight, µg/g. As in the extract of soybean roots (Comparative Example 2), in the root extracts of leguminous plants other than soybeans, the soyasaponins of group DDMP were detected in addition to the soyasaponins of groups B and E.

**[Table 11]**

| µg/g | Kidney bean | Broad bean | Chickpea | Lucerne |
|---|---|---|---|---|
| Soyasaponin I | 67.8±10.6 | 1219.0±137.5 | 831.3±129.4 | 553.8±123.7 |
| Soyasaponin II | 0.0±0.0 | 6.5±4.3 | 0.0±0.0 | 21.8±12.8 |
| Soyasaponin V | 8.8±0.4 | 0.0±0.0 | 0.0±0.0 | 1.5±1.5 |
| Soyasaponin A1 | 0.0±0.0 | 0.0±0.0 | 0.0±0.0 | 0.0±0.0 |
| Soyasaponin A2 | 0.0±0.0 | 0.0±0.0 | 0.0±0.0 | 0.0±0.0 |
| Dehydrosoyasaponin I | 49.6±2.9 | 138.6±19.7 | 321.4±67.1 | 263.7±73.0 |
| Daidzein | 38.0±10.3 | 0.3±0.3 | 1.8±0.5 | 0.3±0.0 |
| Genistein | 4.6±2.0 | 0.5±0.1 | 22.2±9.4 | 4.9±1.4 |
| Glycitein | 1.1±0.1 | 0.0±0.0 | 0.0±0.0 | 0.0±0.0 |
| Daidzin | 2829.2±400.9 | 0.0±0.0 | 1.9±0.6 | 0.0±0.0 |
| Genistin | 74.5±14.6 | 0.0±0.0 | 6.1±3.1 | 0.0±0.0 |
| Glycitin | 0.0±0.0 | 0.0±0.0 | 0.0±0.0 | 0.0±0.0 |
| Malonyldaidzin | 11.2±1.2 | 0.8±0.8 | 8.9±5.5 | 20.4±1.2 |
| Malonylgenistin | 0.8±0.1 | 0.0±0.0 | 116.9±53.5 | 0.0±0.0 |
| Malonylglycitin | 0.0±0.0 | 0.0±0.0 | 0.0±0.0 | 0.0±0.0 |
| Soyasaponin βg* | 70.6±22.3 | 1551.3±245.1 | 94.0±53.6 | 1022.6±222.0 |
| Soyasaponin βa* | 0.0±0.0 | 13.8±6.9 | 6.4±6.4 | 41.6±13.2 |
| Soyasaponin αa* | 0.0±0.0 | 13.5±6.7 | 0.0±0.0 | 120.2±24.0 |
| Soyasaponin γg* | 72.9±26.0 | 41.9±6.3 | 19.8±0.2 | 174.3±54.0 |

| | | | | |
|---|---|---|---|---|
| *: Group DDMP soyasaponin (approximation) | | | | |

**[Table 12]**

| µg/g | Pea | Mung bean |
|---|---|---|
| Soyasaponin I | 2146.5±105.9 | 1542.3±200.5 |
| Soyasaponin II | 33.0±5.6 | 19.5±5.3 |
| Soyasaponin V | 0.0±0.0 | 0.0±0.0 |
| Soyasaponin A1 | 0.0±0.0 | 0.0±0.0 |
| Soyasaponin A2 | 0.0±0.0 | 0.0±0.0 |
| Dehydrosoyasaponin I | 904.9±12.0 | 299.1±67.0 |
| Daidzein | 0.0±0.0 | 40.8±8.2 |
| Genistein | 0.0±0.0 | 1.9±0.2 |
| Glycitein | 0.0±0.0 | 2.3±0.1 |
| Daidzin | 0.0±0.0 | 696.4±169.3 |
| Genistin | 0.0±0.0 | 24.7±1.1 |
| Glycitin | 0.0±0.0 | 0.0±0.0 |
| Malonyldaidzin | 0.0±0.0 | 3.9±0.5 |
| Malonylgenistin | 0.0±0.0 | 0.4±0.1 |
| Malonylglycitin | 0.0±0.0 | 0.0±0.0 |
| Soyasaponin βg* | 2465.4±368.3 | 1460.1±178.1 |
| Soyasaponin βa* | 20.9±0.7 | 21.4±0.7 |
| Soyasaponin αa* | 23.4±1.2 | 23.6±1.4 |
| Soyasaponin γg* | 62.7±8.7 | 72.0±26.6 |

| | | |
|---|---|---|
| *: Group DDMP soyasaponin (approximation) | | |

## Claims

1. A method for producing soyasaponins comprising:
bringing at least a part of a plant body of a leguminous plant into contact with an aqueous solution while culturing or cultivating the plant body using the aqueous, solution; and
collecting the aqueous solution or separating the soyasaponins from the aqueous solution.

2. The method according to claim 1, wherein the culture or cultivation is performed by hydroponic cultivation, spray cultivation, sand cultivation, gravel cultivation, or drip fertigation.

3. The method according to claim 1 or 2, wherein the soyasaponins are at least one selected from the group consisting of glycosides of which aglycone is soyasapogenol B, glycosides of which aglycone is soyasapogenol E, and glycosides of which aglycone is soyasapogenol A.

4. The method according to any one of claims 1 to 3, wherein the mass ratio of soyasaponins to isoflavones in the collected aqueous solution or a product obtained by the separation is 2.0 or more.

5. The method according to any one of claims 1 to 4, wherein the leguminous plant is at least one selected from the group consisting of Glycine plants, Vigna plants, Pisum plants, Medicago plants, and Vicia plants.

6. The method according to any one of claims 1 to 3, wherein the leguminous plant is at least one selected from the group consisting of Cicer plants and Phaseolus plants.

7. A soyasaponins-containing solution produced by the following steps of bringing at least a part of a plant body of a leguminous plant into contact with an aqueous solution while culturing or cultivating the plant body using the aqueous solution and collecting the aqueous solution.

8. The soyasaponins-containing solution according to claim 7, wherein the culture or cultivation is performed by hydroponic cultivation, spray cultivation, sand cultivation, gravel cultivation, or drip fertigation.

9. The soyasaponins-containing solution according to claim 7 or 8, wherein the soyasaponins are at least one selected from the group consisting of glycosides of which aglycone is soyasapogenol B, glycosides of which aglycone is soyasapogenol E, and glycosides of which aglycone is soyasapogenol A.

10. The soyasaponins-containing solution according to any one of claims 7 to 9, wherein the mass ratio of soyasaponins to isoflavones is 2.0 or more.

11. The soyasaponins-containing solution according to any one of claims 7 to 10, wherein the leguminous plant is at least one selected from the group consisting of Glycine plants, Vigna plants, Pisum plants, Medicago plants, and Vicia plants.

12. The soyasaponins-containing solution according to any one of claims 7 to 11, wherein the contents of soyasaponin βg, soyasaponin βa, soyasaponin αa, and soyasaponin yg are each less than 0.05 µg/L.
